# EUROPEAN PATENT SPECIFICATION

(11) **EP 1 300 124 B1**
(45) Date of publication and mention of the grant of the patent: **21.03.2012**
(21) Application number: 02021348.4
(22) Date of filing: 23.09.2002
(51) Int. Cl.: A61F 13/496

(54) **Shorts type disposable diaper**
Wegwerfwindel in Höschenform
Couches-culottes jetables

(30) Priority: 28.09.2001 JP 2001303859; 28.09.2001 JP 2001303860
(43) Date of publication of application: 09.04.2003
(73) Proprietor: KAO CORPORATION, Chuo-Ku, Tokyo (JP)
(72) Inventor: Takei, Shinobu, c/o Kao Corporation, Ichikai-machi, Haga-gun, Tochigi (JP); Ando, Kenji, c/o Kao Corporation, Ichikai-machi, Haga-gun, Tochigi (JP); Hayase, Toru, c/o Kao Corporation, Ichikai-machi, Haga-gun, Tochigi (JP)
(74) Representative: Vossius & Partner

(56) References cited:
- EP-A- 0 626 161
- EP-A- 1 155 668
- EP-A- 1 208 825
- US-A- 5 147 487

## Description

This invention relates to a shorts type disposable diaper which is excellent in wearing feel and anti-leakage property, which can offer a nice looking feature, and which is easy to manufacture. Also, the present invention relates to a shorts type disposable diaper which can be exchanged in a sanitary manner after discharge of feces, which is easy to wear and good in wearing feel, which is excellent in absorption property and anti-leakage property, and which can offer a nice looking feature.

The shorts type disposable diaper is widely used by babies of the high month age, grown-up incontinent persons, and the like. Such a shorts type disposable diaper can easily be worn by a wearer in a manner just like an undergarment shorts. Therefore, many attempts have been made to design a diaper so that it can offer a good wearing feel of the sort which an undergarment can offer. On the other hand, it is necessary for the shorts type disposable diaper to have basic properties (such as absorptive property, anti-leakage property, etc.) which a typical diaper has. In general, elastic members are arranged, for the purposes to improve wearing feel and anti-leakage property, in such a manner as to form gathers around the waist opening portion and the leg opening portion in the shorts type diaper. In particular, since the elastic member disposed around the leg opening portion is located near the urine discharging area and the feces discharging area, it undertakes an important role for anti-leakage.

As a diaper having an elastic member disposed around the leg opening portion, a diaper (for example, Japanese Patent Application Laid-Open No. H02-4364) is known in which an elastic member is disposed in parallel with the longitudinal direction of the diaper and another diaper (for example, Japanese Patent Application Laid-Open No. H57-117602) is also known in which a single elastic member is disposed in a round pattern along the contour of each of a pair of leg opening portions. The former has such a shortcoming that since the elastic member is not disposed along the leg opening portion, the performance is insufficient, while the latter has such a shortcoming that manufacturing efficiency is not completely satisfactory.

A leg elastic member in a typical shorts type diaper is disposed along the leg opening portion. As a known arrangement pattern of such a leg elastic member, the elastic member is disposed, form the view point of manufacture, on each widthwise side of the diaper, in such a manner as to extend from the stomach side portion to the back side portion via the under-crotch portion of the diaper.

A technique (for example, Japanese Patent Application Laid-Open No. H06-296643) is also known in which separate elastic members, one for the stomach side portion and the other for the back side portion, are disposed along one leg opening portion and also disposed along the other leg opening portion after the respective elastic members are traversed in the widthwise direction at the under-crotch portion. This is easy to manufacture and good in efficiency. Although a disposable diaper manufactured according to this conventional technique has such an advantage that a sense of fitness at the under-crotch area is good because the under-crotch portion, when in wear, is pulled up to the stomach side portion and the back side portion, it has such disadvantages that a sense of oppression is given to the under-crotch portion of the wearer and that if the absorbent core at the under-crotch portion is overly contracted in the widthwise direction, absorption efficiency is degraded. Moreover, since the diaper is readily bent at the area where the elastic member is applied due to rigidity of the elastic member, it gives rise to such an additional disadvantage that a pocket having a favorable anti-leakage property is difficult to form.

In case the absorbent core is high in rigidity, the absorbent core at the under-crotch portion is contracted to thereby degrade the absorption efficiency. Moreover, the diaper is readily bent in a protruding fashion towards the wearer's body side at the area where the absorbent core is provided due to rigidity of the absorbent core, thus making it difficult to form, when in wear, a pocket shape which is useful for anti-leakage property and making it difficult to perform, after discharge of feces, a post-treatment easily and in a sanitary manner.

Moreover, there is a still additional disadvantage in that in case the diaper has elastic expanding and contracting properties at the under-crotch region, the absorbent core is contracted in the widthwise direction at this specific area, thus making it difficult to utilizing the absorbent core effectively.

Japanese Patent Application Laid-Open No. H06-197925 discloses a method for manufacturing a disposable diaper, in which after a leg elastic member is bonded to the top of a diaper forming a web, the elastic member is cut at the area corresponding to a central portion of the under-crotch portion so as to form an annular leg elastic region around the leg opening portion.

However, the above-mentioned Laid-Open publication does not disclose the shape of the leg elastic member after cutting in detail. Moreover, at the time of bonding the leg elastic member to the web, the elastic member disposed from the stomach side portion towards the under-crotch portion and the elastic member disposed from the back side portion towards the under-crotch portion are intersected with each other at the under-crotch portion and thereafter, they are brought greatly away from each other. Those two elastic members are in the shape of an open mouth. Accordingly, at the time of cutting those elastic members, there is a high possibility that other sheet member(s) is cut or gets damaged. Moreover, it can be consider that the raising effect at the side portion of the diaper is small and the nearby area of the central portion of the under-crotch portion is greatly swollen in a developed state of the diaper after use, thus making it difficult to treat the discharged feces.

Japanese Patent Application Laid-Open no. 2000-300602 discloses a disposable diaper in which leg elastic members are arranged from four directions of front, rear, right and left towards a central portion of the under-crotch portion of the diaper, and the respective elastic members are not intersected with each other. In this diaper, since no annular elastic region is formed at the leg opening portion, there is a fear that leakage may occur.

It is, therefore, one object of the present invention to provide a shorts type disposable which can be manufactured easily and efficiently, which is easy to wear and good in wearing feel, which is excellent in absorption property and anti-leakage property, and which can offer a nice looking feature.

The invention is defined in the claims.

The present invention has achieved the first object by providing a shorts type disposable diaper comprising a liquid permeable topsheet, a liquid impermeable backsheet and a liquid retentive absorbent core, and having a waist opening portion and a pair of leg opening portions, the leg opening portion having a leg elastic region formed annularly along a peripheral edge thereof, the leg elastic region including a plurality of first elastic members arranged from a stomach side portion to an under-crotch portion and a plurality of second elastic members arranged from a back side portion to the under-crotch portion, the first and second elastic members including an annular portion forming portion for forming the leg elastic region and an extension portion extending from the annular portion forming portion towards a widthwise center of the diaper, the respective extension portions of the first and second elastic members including an end portion at a location where the extension portions do not reach the widthwise center of the diaper, the extension portions being designed so as not to exhibit elastic contractibility in a vicinity of the end portion, the extension portions of the first and second elastic members being arranged such that one elastic member and the other elastic member are overlapped.

The present invention also provides a method for manufacturing a shorts type disposable diaper having a first and a second elastic member which are arranged along peripheral edges of a pair of leg opening portions and which are not arranged at a widthwise central portion of an under-crotch portion said method comprising the steps of:
introducing the first and second elastic members in their stretched states onto a base material sheet which is continuously conveyed while swaying the first and second elastic materials in a direction orthogonal to a flowing direction of the base material sheet and thereafter cutting the first and second elastic members at an under-crotch central portion corresponding to a widthwise central portion of an under-crotch portion of the diaper,
the first and second elastic members being introduced in a state where one elastic member and the other elastic member are overlapped one upon the other at the under-crotch central portion and the overlapped elastic members being pressed and cut in a thickwise direction by a cutting protrusion.

The present invention will be more particularly described with reference to the accompanying drawings, in which:
Fig. 1 is a perspective view showing one embodiment of a shorts type disposable diaper of the present invention ;
Fig. 2 is a plan view showing a developed and stretched state of the disposable diaper shown in Fig. 1;
Fig. 3 is a partly enlarged plan view showing one form of arrangement of an elastic member at an under-crotch portion of the disposable diaper shown in Fig. 1, together with a sectional view taken on line X-X of this plan view;
Fig. 4 is a perspective view showing a state (standstill state) in which the disposable diaper shown in Fig. 1 is developed and stationarily placed on a flat horizontal plane;
Fig. 5 is a schematic sectional view taken on line Y-Y of Fig. 4;
Fig. 6 is a perspective view showing one embodiment of a method for manufacturing a shorts type disposable diaper of the present invention;

The present invention will be described hereinafter with reference to the preferred embodiments.

First, one embodiment of a shorts type disposable diaper of the invention will be described.

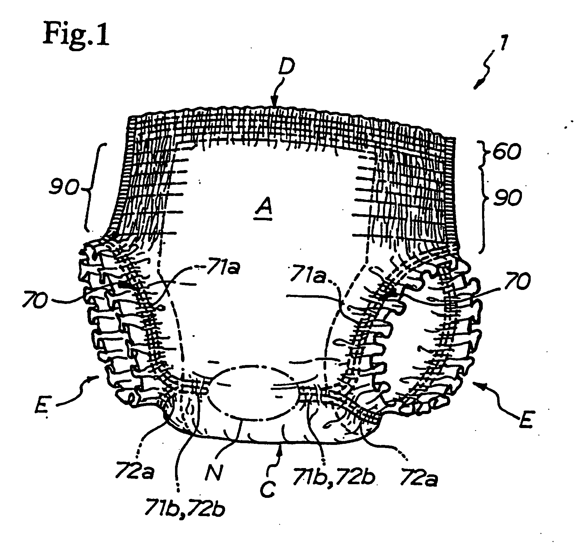

A shorts type disposable diaper 1 shown in Figs. 1 through 3 is a disposable diaper which comprises a liquid permeable topsheet 2, a liquid impermeable backsheet 3 and a liquid retentive absorbent core 4 interposed therebetween and in which opposite side edge portions of a stomach side portion A and opposite side edge portions of a back side portion B are joined together to form a waist opening portion D and a pair of leg opening portions E, E.

In the disposable diaper 1 of this embodiment, the absorbent core 4 is in a hour grass shape whose central portion constricted. The topsheet 2 and the backsheet 3 are also in the same shape in match with the outer contour of the absorbent core 4. The topsheet 2 and the backsheet 3 are joined together at the outside of opposite side edges in the longitudinal direction of the absorbent core 4 and also jointed together at the portions extending from the longitudinal side edges of the absorbent core 4.

Two outer layer nonwoven fabrics 51, 52 are disposed at an outer side (outer surface side of the diaper) of the backsheet 3. The outer layer nonwoven fabrics extend outward of the diaper beyond longitudinal opposite ends and opposite side edges of the absorbent core 4. A peripheral edge portion of the waist opening portion D and peripheral edge portions of the leg opening portions E, E are formed by the extended outer layer nonwoven fabrics. A waist elastic member 6, leg elastic members 71, 72 and a girth elastic member 9 are disposed between the two outer layer nonwoven fabrics 51, 52 at a prescribed part to thereby form a waist elastic region (waist gather) 60, an annular leg elastic region (leg gather) 70 and a girth elastic region (girth gather) 90. The topsheet 2, the backsheet 3 and the absorbent core 4 are integrated to form an absorptive main body 10. The absorptive main body 10 is fixedly joined to an outer layer body 5 comprising the two outer layer nonwoven fabrics 51, 52. A three-dimensional gather 8 is formed along each opposite side edges of the absorbent core 4.

The leg elastic region (leg gather) 70 in the disposable diaper 1 of this embodiment is formed in an annular shape which is closed along the peripheral edge of the leg opening portion E. Specifically, it comprises a plurality of first elastic members 71 arranged from the stomach side portion A to the under-crotch portion C and a plurality of second elastic members 72 arranged from the, back side portion B to the under-crotch portion C.

The first and second elastic members 71, 72 include annular portion forming portions 71a, 72a disposed along the peripheral edges of the leg opening portions and adapted to form a leg elastic region 70, and extension portions 71b, 72b extending from the annular portion forming portions 71a, 72a towards a widthwise center (center line for widthwise bisecting the diaper) of the diaper, respectively. The extension portions 71b, 72b of the first and second elastic members include end portions 71c, 72c at locations where the extension portions do not reach the widthwise center (center line for widthwise bisecting the diaper) of the diaper, respectively. The extension portions 71b, 72b are released from their stretched states in the nearby areas of the end portions so that no elastic contractibility is exhibited. In the extension portions 71b, 72b of the first and second elastic members 71, 72, one elastic member 71 and the other elastic member 72 are overlapped one upon the other.

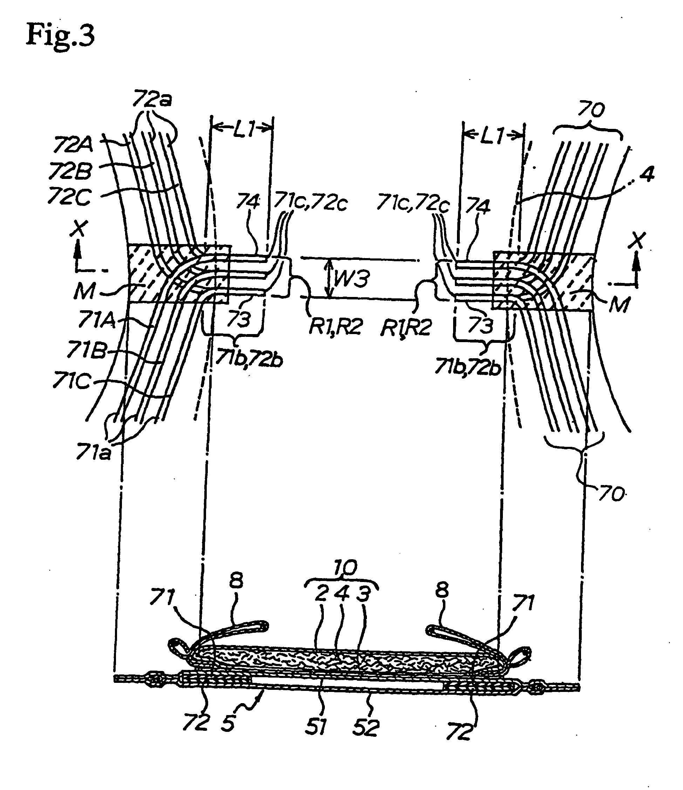

That is, in the diaper 1 of this embodiment, as shown in Fig. 3, a plurality of first and second elastic members are disposed in their overlapped states (states where one elastic members 71 and the other elastic members are overlapped one upon the other) at the under-crotch portion C. The portions where the two elastic members are overlapped form the extension portions 71b, 72b of the first and second elastic members.

More specifically, a plurality (three in the illustrated example) of the first and second elastic members 71, 72 are arranged. The first elastic members 71 themselves are not overlapped one upon the other, and the second elastic members 72 themselves are not overlapped one upon the other, either.

Still more specifically, the first and second elastic members 71, 72 are each in a belt-like configuration. At the under-crotch portion C, as shown in Fig. 3, an elastic member 71A whose annular portion forming portion (gather forming portion) 71a exists at an outermost side of the leg opening portion (peripheral edge end side) of all the three first elastic members is intersected by an outermost side elastic member 72A and a middle elastic member 72B of the second elastic members, the most outer side elastic member 72A of the second elastic members is converged and overlapped with an innermost side elastic member 71C of the first elastic members, and a middle elastic member 72B of the second elastic members is converged and overlapped with a middle elastic member 71B of the first elastic members. On the other hand, a most inner side elastic member 72C of the second elastic members is converged and overlapped with a most outer side elastic member 71A of the first elastic members. The portions where 71A and 72C, 71B and 72B, and 71C and 72A are overlapped with each other, respectively, form the extension portions 71b, 72b of the respective elastic members.

Let's presume here that the first elastic members 71A through 71C are regarded as a single belt and the second elastic members 72A through 72C are regarded as a single belt and those belts are viewed from the widthwise outside of the diaper towards the inside. Then, the expression "annular portion forming portion" used in this embodiment refers to a portion, which is located at a widthwise outer side of the diaper from a part where two belts are completely overlapped for the first time, and the expression "extension portion" refers to a portion which is located at a widthwise inner side of the diaper from the above-mentioned completely overlapped portion.

By providing a plurality of the first and second elastic members 71, 72 in such manner as to form such extension portions 71b, 72b, the annular leg elastic region 70, which is closed along the peripheral edge of the leg opening portion, can be formed. Moreover, the first and second elastic members become difficult to escape. Accordingly, when in wear, the leg elastic region (leg opening portion gather) can be made to act in a stable manner.

Moreover, since the extension portions 71b, 72b of the first and second elastic members 71, 72 each include an end portion at a location where the extension portions do not reach the widthwise center of the diaper, it never happens that the wearing feel is degraded by oppressing the wearer's under-crotch portion. Moreover, since the absorbent core is hardly contracted in the widthwise direction, liquid dispersion is not prevented within the absorbent core. Since twisting and breakage hardly occur, absorption efficiency of the absorbent core can be prevented from lowering. There is no provision of the elastic members 71, 72 at the widthwise central area of the diaper which is indicated by reference character N in Fig. 1.

Since it is designed such that the end portions 71c, 72c of the first and second elastic members 71, 72 on the widthwise center side of the diaper do not exhibit elastic contractibility, it never happens that the wearer's under-crotch portion is oppressed and the absorbent core is contracted in the widthwise direction even in such an embodiment that the first and second elastic members 71, 72 are overlapped with the absorbent core 4 at a side portion of the absorbent core 4.

Since the nearby areas of the end portions 71c, 72c of the first and second elastic members 71, 72 on the widthwise center side of the diaper do not exhibit elastic contractibility, the following additional effects can be obtained.
(1) In case a region exhibiting no elastic contractibility in the nearby areas of the end portions 71c, 72c is located only at the inner side from the side edge of the absorbent core, the side portion of the absorbent core is pulled up to the wearer's inguinal part. Accordingly, a wall of the absorbent core is formed on a side surface having a pocket-like configuration and anti-leakage property is enhanced especially when urine discharging speed is fast.
(2) In case the region exhibiting no elastic contractibility in the nearby areas of the end portions 71c, 72c is located only at the outer side from the side edge of the absorbent core or in case it is located from the inner side of the absorbent core to the outer side, the pocket-like configuration to be formed can have a wide dimension. Accordingly, anti-leakage property is enhanced especially when an amount of discharged urine and an amount of discharged feces is large.

Since the first and second elastic members 71, 72 are arranged such that one elastic member 71 is overlapped with the other elastic member 72 at the extension portions 71b, 72b, an appropriate degree of rigidity is given to the arrangement region R1 of the first elastic member and the arrangement region R2 of the second elastic member at the side portion of the under-crotch portion of the diaper and at the side portion of the absorbent core 4, and at the same time, only the opposite side portions of the absorbent core 4 are pulled up to the wearer's inguinal portion. Accordingly, the absorbent core is not contacted in the widthwise direction and in that condition, a pocket-like configuration protruding to the opposite side of the wearer's body side is formed. Thus, since shape retentive property is enhanced, anti-leakage property is enhanced, too.

In case the absorbent core forms a pocket-like configuration protruding to the opposite side of the wearer's body side, the absorbent core is not prevented from swelling after urine is discharged. Moreover, since the swollen absorbent core is not intimately adhered to the wearer's body, the shape of the pocket can be maintained for a long time. Accordingly, high anti-leakage property can be maintained even after urine and feces are discharged several times.

As a synergetic effect of the above-mentioned construction and a construction in which no elastic member is disposed at the widthwise center of the diaper and the end portions of the elastic members do not exhibit elastic expansibility and contractibility, a pocket configuration having a wide and deep excretion discharging point is formed, and therefore, excretion is hardly leaked in the widthwise direction when the diaper is put off by detaching the joint portion at the side edge in order to make a diaper exchange after excretion is discharged. Moreover, at the time of cleaning the wearer's hip by raising the hip after the diaper developed under the wearer's hip is spread, the absorbent core is not greatly bent towards the wearer's body side. Therefore, the absorbent core does not interfere the cleaning work and excretion does not adhere to the wearer.

In case the first and second elastic members 71, 72 are not overlapped with each other at their extension portions (as shown e.g., in EP-A-1 155 668), a total (six) of the number (for example, three) of the first elastic members and the number (for example, three) of the second elastic members can be visually recognized at the under-crotch portion. In this embodiment, since the number of the elastic members, which can visually be recognized at the under-crotch portion is a half (three) the number which is actually used, the outer appearance is simple and yet a nice looking feature can be exhibited.

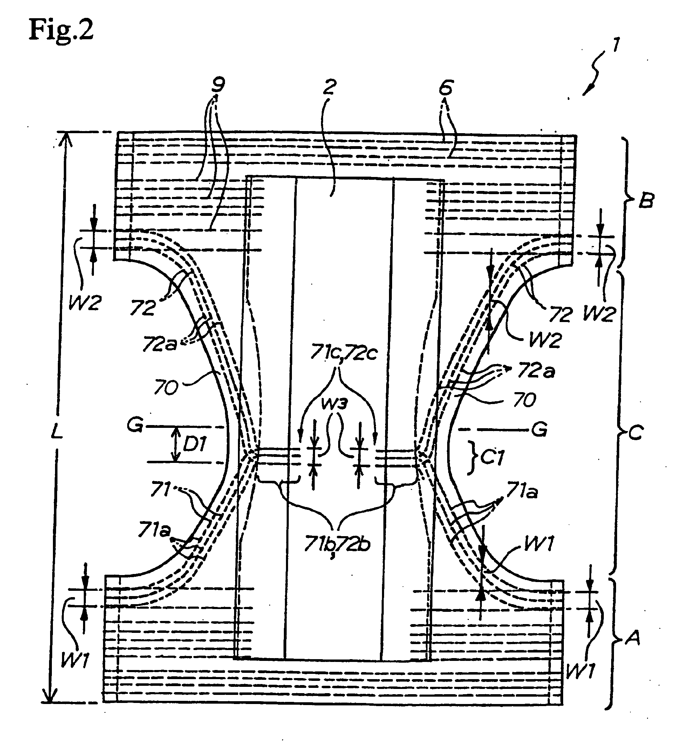

In this embodiment, the extension portions 71b, 72b of the first and second elastic members are located more on the stomach side portion A side from the longitudinal center (center line for bisecting the longitudinal direction, G-G line of Fig. 2) of the diaper in a state (see Fig. 2) in which the disposable diaper is developed and stretched.

The degree of displacement towards the stomach side portion A side is preferably as follows. In the developed and stretched state of the diaper (see Fig. 2), a distance D1 between the edge portion of the elastic member which is located at the most stomach side portion side and the longitudinal center is preferably 15 % or less with respect to the entire length L in the longitudinal direction, and more preferably 10 % or less.

In case the arrangement region R1 of the first elastic member and the arrangement region R2 of the second elastic member are not overlapped with each other, absorption performance is disadvantaged to the extent of the interval between the region R1 and the region R2. Moreover, in order to have a wide elastic member traversing region in the longitudinal direction, one elastic member is unnecessarily disposed at the stomach side portion or at the central portion of the under-crotch portion. Accordingly, there is such a fear that wearing feel is sacrificed.

The first and second elastic members 71, 72 in the disposable diaper 1 of this embodiment are fixed at the leg elastic region 70 (see Fig. 2, Fig. 3) to an adjacent outer layer nonwoven fabric (sheet material) by first fixing means (adhesive agent) and in the vicinity M of the boundary between the annular portion forming portions 71a, 72a and the extending portions 71b, 72b to the adjacent outer layer nonwoven fabric (sheet material) by second fixing means (adhesive agent) which is applied to a smaller range than the first fixing means or by the first fixing means and the second fixing means which is applied to a smaller range than the first fixing means.

In case the first fixing means is applied over the entire area of the sheet material which is adjacent to the elastic member and by this, the elastic member is completely fixed to the sheet material, even if attempt is made to cut the elastic member into a left and a right part after the elastic member is disposed across the under-crotch portion, the elastic member still capable of exhibiting elastic expansibility and contractibility keeps remaining at the central portion of the under-crotch portion. Accordingly, there is such a fear that the effect of the present invention is unable to obtain. Although there can be contemplated that adhesive agent is annularly applied only to the leg elastic region, there is possibility of an occurrence of rubber coming-off at the leg opening portion because the leg opening portion is occasionally pulled hard in response to vigorous motion of the wearer and thus, adverse effect resulting from the releasing of the stretching state of the elastic member cut off at the central portion of the under-crotch portion may prevail on the elastic member arranged along the edge portion of the leg opening portion.

If the first and second elastic members are fixed in the manner as in this embodiment, rubber coming-off can be prevented from occurring while prohibiting the exhibition of elastic expansibility and contractibility at the central portion of the under-crotch portion.

Moreover, by applying the second fixing means in a pattern different from that of the first fixing means, stiffness and sense of non-flexibility resulting from overly applied adhesive agent can be prevented from occurring at other regions. Thus, there is such an advantage that texture of the diaper is not sacrificed.

The first fixing means is for fixing the entire or a part of the annular portion forming portion (gather forming portion) of the first and second elastic members, and the second fixing means is applied to a smaller range of area than the first fixing means. The range of area where the first or second fixing means is applied includes, for example, an area between adjacent dots in case adhesive agent is applied in a dot pattern.

A part M where the second fixing means (adhesive agent) is applied may be a part in which the stretching state of the elastic member which has been cut off is released at least in the vicinity of the cut-off end portion and the effect of such releasing is prohibited from prevailing on the part disposed along the edge portion of the leg opening portion. Moreover, by applying the second fixing means to both the areas between the elastic member and one of two sheet materials adjacent thereto and between the elastic member and the other sheet material, the effect of releasing of the stretching state can more effectively be prevented from prevailing on the annular portion forming portion. Moreover, examples of a combination of the first fixing means and the second fixing means may include, among others, a first example in which the first fixing means is an adhesive agent and the second fixing means is a same or different kind of adhesive agent and a second example in which the first fixing means is an adhesive agent and the second fixing means is heat sealing.

According to the diaper 1 of this embodiment, a maximum distance (maximum distance measured along the longitudinal direction of the diaper) in the longitudinal direction of the diaper between the stomach side portion side edge portion 73 of the extension portions 71b, 72b located at the most stomach side portion side in the longitudinal direction of the diaper and the back side potion side edge portion 74 of the extension portion of the first or second elastic member located at the most back side portion side in the longitudinal direction of the diaper in a developed and stretched state (state in which the diaper is developed and the elastic members at various parts are stretched, see Fig. 2) of the diaper, is smaller than a sum (W1 + W2) of a maximum arrangement width in the longitudinal direction of the diaper in the annular portion forming portion (gather forming portion) of a plurality of the fist elastic members and a maximum arrangement width in the longitudinal direction of the diaper in the annular portion forming portion (gather forming portion) of a plurality of the second elastic members.

The expression "the maximum arrangement width W1 (W2) of a plurality of the first (second) elastic members" refers to a maximum value of the arrangement width obtained by measuring a distance between outer edges of the elastic members located at the outer sides of the first (second) elastic members in a developed and stretched state of the diaper along the longitudinal direction (up and down direction of Fig. 2) of the diaper. The first and second elastic members 71, 72 in this embodiment are constant in arrangement width W1 (W2) over the entire area in the widthwise direction of the diaper.

According to the diaper of this embodiment, since the maximum distance W3 in the longitudinal direction of the diaper between the stomach side portion side edge portion 73 of the extension portion of the first or second elastic member located at the most stomach portion side and the back side portion side edge portion 74 of the first or second elastic member located at the most back side portion side in the developed and stretched state (see Fig. 2) of the diaper, is smaller than the sum (W1 + W2) of the maximum arrangement width, in the longitudinal direction of the diaper, of the fist and second elastic members 71, 72, the diaper in its developed state, especially, its absorbent core can be prevented from being greatly bent in the longitudinal direction caused by rigidity of the elastic members.

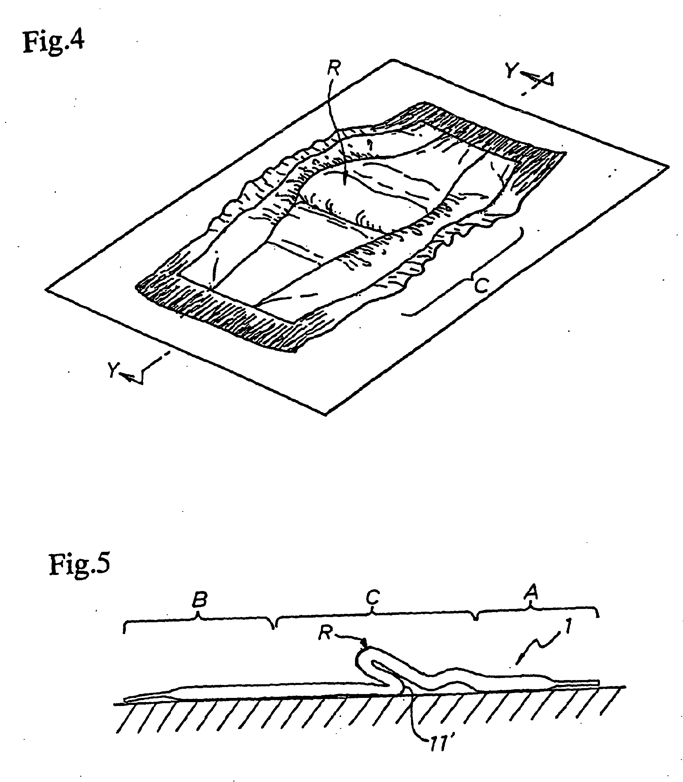

That is, in a shorts type disposable diaper, the shape of the shorts type is usually formed by folding back the central portion of the under-crotch portion (nearby area of the center line for bisecting the longitudinal direction) and joining the side edge portions located between the leg opening portion and the waist opening portion. For this reason, as shown in Figs. 4 and 5, a folding portion 11' having a folding habit is formed at the central portion of the under-crotch portion. Therefore, when the diaper is developed and placed on a plane, the absorbent core is raised upward at its front or rear part in the longitudinal direction of the diaper with reference to the folding portion 11', thereby a raised portion R in which a part having the absorbent core 4 arranged thereon is partly raised upward is formed on the under-crotch portion C of the diaper.

At the time of diaper exchange, since the wearer's hip is cleaned by raising lifting the hip upward, load is applied to a rear half part (portion of the waist opening portion offset to the end edge) in the back side portion of the diaper in its developed state. For this reason, in case the high raised portion is located, in the natural state (that is, state where no load is applied in a developed state), at the back side of the diaper, the stomach side of the diaper is, when the raised portion is pressed by the wearer, pulled towards the wearer side, thereby interfering the diaper exchanging work. In such a case, since inclination is directed to the wearer side, there is such a fear that excretion, if this being soft feces, migrates towards the wearer side. On the other hand, in case the raised portion is, in the natural state, located at the stomach side of the diaper, the above-mentioned problem hardly occurs but the wearer's hip is difficult to clean.

In the disposable diaper 1 of this embodiment, since the maximum distance W3 is set to be smaller than the sum (W1 + W2) of the maximum arrangement widths, the above-mentioned problem can be solved.

From a view point of conducting a diaper exchanging work in a sanitary manner and with ease, and of exhibiting the effect of anti-leakage as later described, a ratio [W3/(W1 + W2)] between the maximum distance W3 and the sum (W1 + W2) of the maximum arrangement widths is preferably 0.3 to 0.95 and particularly preferably 0.4 to 0.7. From the same view point, the maximum distance W3 is preferably 5 to 60 mm, and the sum (W1 + W2) of the maximum arrangement widths is preferably 10 to 80 mm. Similarly, the maximum arrangement width W1 of the first elastic members is preferably 5 to 40 mm, and the maximum arrangement width W2 of the second elastic members is preferably 5 to 40 mm.

In the diaper of this embodiment, since the region where the first elastic members are disposed and the region where the second elastic members are disposed are overlapped with each other at the under-crotch portion, the overlapped portion is more increased in rigidity. Accordingly, a provision of such elastic members across the under-crotch portion tends to bend the diaper, especially the absorbent core easily. However, as mentioned above, since the first and second elastic members 71, 72 are not allowed to extend up to the widthwise center of the diaper, the above-mentioned problem can be prevented from occurring.

Moreover, as mentioned above, since the extension portions 71b, 72b of the first and second elastic members 71, 72 are located at the side portion of the absorbent core 4 in their overlapped states, there can be obtained a high rigidity owing to the arrangement in which the arrangement region of the first elastic members and the arrangement region of the second elastic members are overlapped with each other while preventing the absorbent core 4 from contacting in the widthwise direction. By virtue of this high rigidity accompanying with the effect for pulling the first and second elastic members towards the stomach side and the back side when the diaper is in wear, the opposite sides of the absorbent core 4 are pulled upward from the wearer's under-crotch portion to form a pocket. Accordingly, the anti-leakage effect is further enhanced. Moreover, owing to this arrangement, the excretion discharging point is wide and the pocket shape is formed.

Accordingly, there is almost no fear that excretion leaks in the widthwise direction at the time of detaching the joint portion of the side edges when diaper exchange is conducted at the time of excretion discharge.

Moreover, since the absorbent core 4 is restrained in contacting in the widthwise direction, there can be obtained a neat outer appearance, as well as a nice-looking appearance.

Moreover, the absorbent core 4 is not greatly contracted in the widthwise direction at the under-crotch region (the area, as well its nearby area, where the extension portions 71b, 72b are located at the opposite side portions) C1. In addition, a tensile force is concentrically applied to the terminals (nearby area of the boundary portion between the annular portion forming portion and the extension portion) at the under-crotch portion of the first and second elastic members 71, 72 which are disposed at the generally annular leg elastic region.

For this reason, the central portion of the absorbent core tends to form a protruding configuration in a direction opposite to the wearer side, and the opposite side portions of the absorbent core are readily raised towards the wearer side at the absorbent core side portion. That is, since a pocket structure is naturally formed at the under-crotch portion, a high anti-leakage property can be exhibited.

Moreover, in the first and second elastic members 71, 72, the nearby areas of the end portions 71c, 72c at the widthwise center side of the diaper is released their stretched states so as not to exhibit elastic contractibility, and not gathers are formed on the part where the extension portion is disposed.

By this, a proper degree of rigidity is given to this portion, thus enabling to form the pocket structure at the under-crotch portion into an ideal shape. Thus, anti-leakage property is enhanced. Moreover, in case the absorbent core is high in rigidity (for example, in case a taber rigidity value is 10 cN· cm or larger) in spite of that the first and second elastic members are overlapped with each other at the side portion of the absorbent core 4, deterioration in absorbing capability, which would otherwise occur due to deshaping of the absorbent core, can be prevented from occurring.

The above-mentioned effect of the present invention becomes particularly manifest in case the rigidity value of the absorbent core obtained through the Taber stiffness test method (JIS P8125) is 1 to 5cN·cm, and such rigidity is preferable because skin touch for the wearer becomes comfortable.

Next, a method for manufacturing a shorts type disposable diaper of the present invention will be described with reference to a method for manufacturing the disposable diaper of the above-mentioned embodiment.

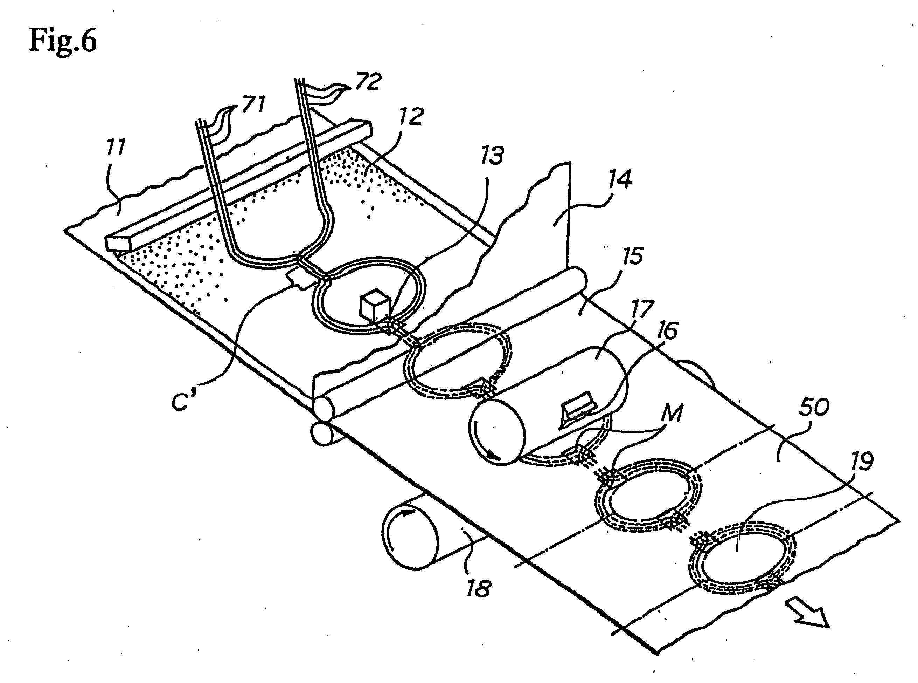

First, as shown in Fig. 6, first and second elastic members 71, 72 are introduced, in their stretched states, onto a continuously conveying base material sheet 11 while swaying the first and second elastic members 71, 72 in a direction orthogonal to the flowing direction of the base material sheet 11. Prior to this introduction, an adhesive agent 12 as the first fixing means is applied to the top of the base material sheet 11 at a width larger than the swaying width of the first and second elastic members.

At the time of introduction of the first and second elastic members 71, 72, the first and second elastic members 71, 72 are linearly disposed at a central portion C' of the under-crotch portion corresponding to the widthwise central portion of the under-crotch portion of the diaper, and the first and second elastic members 71, 72 are overlapped with each other at the linear portion.

Then, an adhesive agent 13 as the second fixing means is intermittently applied to the nearby areas M, M of the boundary portion between the annular portion forming portion 71a, 71a and the extension portions 71b, 72b in the first and second elastic members 71, 72.

Then, another sheet material 14 is overlapped on and adhered to the elastic members 71, 72 which are located on the base material sheet 11 so as to form a laminated body 15. The laminated body 15 is inserted between a cut roll 17 provided at a peripheral surface thereof with a cutting protrusion 16 and an anvil roll 18 place opposite the cut roll 17. Owing to this arrangement, the overlapped first and second elastic members 71, 72 at the central portion C' of the under-crotch portion corresponding to the widthwise central portion of the under-crotch portion of the diaper are pressed in the thickness direction and cut by the protrusion 16.

By this cutting, the portions of the first and second elastic members 71, 72 where only the adhesive agent 12 is applied, are contracted towards the opposite sides of the cutting portion, thereby releasing the stretched states. On the other hand, at the portions M, M where the first and second elastic members 71, 72 are joined to the adjacent base sheet materials 11, 14 by the first and second fixing means, since the first and second elastic members 71, 72 are firmly fixed, releasing of their stretched states is not progressed to the annular portion forming portions 71a, 72a.

In this way, there can be obtained a continuous body 50 of the outer layer body 5. Although not shown, the elastic members for forming the waist elastic region and the girth elastic region are also introduced onto the base sheet material (they are continuously or intermittently introduced by a desired method and subjected to secondary treatment such as cutting, where necessary).

After the absorptive main body 10 is placed and fixed, an excessive portion 19 for forming the leg opening portion is cut off the continuous body 50 of the outer layer body 5 and the continuous body 50 is cut into individual disposable diaper dimensions. Then, the stomach side portion A and the back side portion B are joined to form the shorts type disposable diaper 1 of the above-mentioned embodiment.

According to a method for manufacturing a shorts type disposable diaper of this embodiment, since the elastic member disposed across the under-crotch portion is cut at the portion where the first and second elastic members are overlapped, an amount of deformation of the elastic member is increased and therefore, the elastic member can easily be cut. Moreover, the sheet material adjacent to the elastic member can be restrained from getting damaged, which would otherwise occur in associate with the cutting operation of the elastic member. For this reason, even in the case where the sheet material adjacent to the elastic member is exposed to the outer surface of the diaper and used in that state, the diaper can be prevented from degrading in its nice looking feature. Moreover, the strength can also be prevented from getting lowered.

From the view point of prevention of lowering in strength and getting damage of the sheet material, the cutting protrusions are preferably individually provided (three in the above embodiment) for every elastic member. The rolls 17 and/or 18 may be heated.

The present invention is not limited to the above various embodiments but many changes and modifications can be made.

As materials for forming the various members of the disposable diaper of the present invention, known materials used for the conventional disposable diapers may be used.

The disposable diaper of the invention can easily and efficiently be manufactured, it is easy to wear and good in wearing feel, excellent in absorption efficiency and anti-leakage property, and it can offer a nice looking feature.

## Claims

1. A shorts type disposable diaper comprising a liquid permeable topsheet (2), a liquid impermeable backsheet (3) and a liquid retentive absorbent core (4), and having a waist opening portion (D) and a pair of leg opening portions (E, E),
said leg opening portion (E) having a leg elastic region (70) formed annularly along a peripheral edge thereof, said leg elastic region (70) including a plurality of first elastic members (71) arranged from a stomach side portion (A) to an under-crotch portion (C) and a plurality of second elastic members (72) arranged from a back side portion (B) to said under-crotch portion (C),
said first and second elastic members (71, 72) including an annular portion forming portion (71a, 72a) for forming said leg elastic region (70) and an extension portion (71b, 72b) extending from said annular portion forming portion (71 a, 72a) towards a widthwise center of said diaper, respective said extension portions (71b, 72b) of said first and second elastic members (71, 72) including an end portion (71 c, 72c) at a location where said extension portions (71b, 72b) do not reach the widthwise center of said diaper, said extension portions (71b, 72b) being designed so as not to exhibit elastic contractibility in a vicinity of said end portion (71c, 72c), said extension portions (71b, 72b) of said first and second elastic members (71, 72) being arranged such that pairs of one elastic member (71) and the other elastic member (72) are overlapped, wherein the portions where said one elastic member (71) and said other elastic member (72) overlap with each other, respectively, form the extension portions (71b, 72b) of the respective elastic members (71, 72).

2. The shorts type disposable diaper according to claim 1, wherein said extension portions (71b, 72b) of said first and second elastic members (71, 72) are in position located on the stomach side portion side from a longitudinal center of said diaper in a state where said shorts type disposable diaper is developed and stretched.

3. The shorts type disposable diaper according to claim 1 or 2, wherein said first and second elastic members (71, 72) are, in said leg elastic region (70), fixed to an adjacent sheet material by first fixing means and are, in a vicinity (M) of a boundary between said annular portion forming portion (71a, 72a) and said extension portion (71b, 72b), fixed to adjacent sheet material by second fixing means which is applied to a narrower range than a range to which said first fixing means is applied, or by said first fixing means and said second fixing means.

4. The shorts type disposable diaper according to claim 3, wherein said first and second elastic members (71, 72) are fixed to adjacent two sheet materials in a vicinity of a boundary between said annular portion forming portion (71a, 72a) and said extension portion (71b, 72b).

5. The shorts type disposable, diaper according to claim 1, wherein said extension portions (71b, 72b) of said first and second elastic members (71, 72) are overlapped at least at a part thereof with said absorbent core (4).

6. The shorts type disposable diaper according to claim 1, wherein said first and second elastic members (71, 72) are sandwichingly held in their stretched states between two sheets which are separately formed from said backsheet (3) and disposed at an outer surface side of said backsheet (3).

7. A method for manufacturing a shorts type disposable diaper having a first and a second elastic member (71, 72) which are arranged along peripheral edges of a pair of leg opening portions (E, E) and which are not arranged at a widthwise central portion of an under-crotch portion (C), said method comprising the steps of:
introducing said first and second elastic members (71, 72) in their stretched states onto a base material sheet (11) which is continuously conveyed while swaying said first and second elastic materials (71, 72) in a direction orthogonal to a flowing direction of said base material sheet (11) and thereafter cutting said first and second elastic members (71, 72) at an under-crotch central portion (C') corresponding to a widthwise central portion of an under-crotch portion (C) of said diaper,
said first and second elastic members (71, 72) being introduced in a state where one elastic member (71) and the other elastic member (72) are overlapped one upon the other at the under-crotch central portion (C') and said overlapped elastic members (71, 72) being pressed and cut in a thickwise direction by a cutting protrusion (16).

## Patentansprüche

1. Wegwerfwindel in Höschenform, mit einer flüssigkeitsdurchlässigen oberen Schicht (2), einer flüssigkeitsundurchlässigen hinteren Schicht (3) und einem flüssigkeitszurückhaltenden absorbierenden Kern (4) und mit einem Taillenöffnungsabschnitt (D) und zwei Beinöffnungsabschnitten (E,E),
wobei der Beinöffnungsabschnitt (E) einen ringförmig entlang seiner Randkante gebildeten elastischen Beinbereich (70) aufweist, wobei der elastische Beinbereich (70) mehrere erste elastische Elemente (71), die sich von einem Bauchabschnitt (A) zu einem Schrittabschnitt (C) erstrecken, und mehrere zweite elastische Elemente (72), die sich von einem Rückenabschnitt (B) zu dem Schrittabschnitt (C) erstrecken, aufweist,
wobei die ersten und zweiten elastischen Elemente (71,72) jeweils einen ringabschnittbildenden Abschnitt (71a,72a), der den elastischen Bereich (70) bildet, sowie einen Verlängerungsabschnitt (71b,72b) aufweisen, der sich von dem ringabschnittbildenden Abschnitt (71a,72a) aus hin zur Mitte der Windelbreite erstreckt, wobei jeweilige Verlängerungsabschnitte (71b,72b) der ersten und zweiten elastischen Elemente (71,72) einen Endabschnitt (71c,72c) an einem Ort aufweisen, wo die Verlängerungsabschnitte (71b,72b) die Mitte der Windelbreite nicht erreichen,
wobei die Verlängerungsabschnitte (71b,72b) in der Nähe des Endabschnitts (71c,72c) kein elastisches Zusammenziehvermögen haben,
wobei die Verlängerungsabschnitte (71b,72b) der ersten und zweiten elastischen Elemente (71,72) so angeordnet sind, dass sich Paare aus dem einen elastischen Element (71) und dem anderen elastischen Element (72) überlappen,
wobei die Abschnitte, in denen sich das eine elastische Element (71) und das andere elastische Element (72) überlappen, die Verlängerungsabschnitte (71b,72b) der jeweiligen elastischen Elemente (71,72) bilden.

2. Wegwerfwindel in Höschenform nach Anspruch 1, wobei in einem Zustand, in welchem die Höschen-Wegwerfwindel entfaltet und gedehnt ist, die Verlängerungsabschnitte (71b,72b) der ersten und zweiten elastischen Elemente (71,72) an der Bauchseite der Windel, abseits der Mitte der Windellänge, angeordnet sind.

3. Wegwerfwindel in Höschenform nach Anspruch 1 oder 2, wobei die ersten und zweiten elastischen Elemente (71,72) in dem elastischen Beinbereich (70) mit Hilfe von ersten Befestigungsmitteln an einem benachbarten Schichtmaterial angebracht sind und in einem Grenzbereich (M) zwischen dem ringabschnittbildenden Abschnitt (71a,72a) und dem Verlängerungsabschnitt (71b,72b) mit Hilfe von zweiten Befestigungsmitteln, die auf eine kleinere Fläche aufgebracht sind als die ersten Befestigungsmittel, oder mit Hilfe der ersten Befestigungsmittel und der zweiten Befestigungsmittel an einem benachbarten Schichtmaterial angebracht sind.

4. Wegwerfwindel in Höschenform nach Anspruch 3, wobei in einem Grenzbereich zwischen dem ringabschnittbildenden Abschnitt (71a,72a) und dem Verlängerungsabschnitt (71b,72b) die ersten und zweiten elastischen Elemente (71,72) an zwei benachbarten Schichtmaterialien angebracht sind.

5. Wegwerfwindel in Höschenform nach Anspruch 1, wobei sich die Verlängerungsabschnitte (71b,72b) der ersten und zweiten elastischen Elemente (71,72) zumindest teilweise mit dem absorbierenden Kern (4) überlappen.

6. Wegwerfwindel in Höschenform nach Anspruch 1, wobei die ersten und zweiten elastischen Elemente (71,72) in ihrem gedehnten Zustand zwischen zwei Schichten gehalten werden, die separat aus der hinteren Schicht (3) gebildet sind und an einer äußeren Oberfläche der hinteren Schicht (3) angeordnet sind.

7. Verfahren zur Herstellung von Wegwerfwindeln in Höschenform, die erste und zweite elastische Elemente (71,72) aufweisen, die entlang von Randkanten zweier Beinöffnungen (E,E) angeordnet sind und die nicht in einem der Breite nach zentralen Abschnitt eines Schrittabschnitts (C) angeordnet sind, wobei das Verfahren die folgenden Schritte aufweist:
Aufbringen von ersten und zweiten elastischen Elementen (71,72) in gedehntem Zustand auf ein flächiges Basismaterial (11), das kontinuierlich zugeführt wird, unter Hin- und HerBewegung der ersten und zweiten elastischen Elemente (71,72) in eine zur Laufrichtung des flächigen Basismaterials (11) orthogonale Richtung und danach Schneiden der ersten und zweiten elastischen Elemente (71,72) an einem zentralen Schrittabschnitt (C'), der einem der Breite nach zentralen Abschnitt des Schritts (C) der Windel entspricht,
wobei die ersten und zweiten elastischen Elemente (71,72) so zugeführt werden, dass sich an dem zentralen Schrittabschnitt (C') das eine elastische Element (71) und das andere elastische Element (72) überlappen, und die sich überlappenden elastischen Elemente (71,72) in Richtung der Dicke gepresst werden und mit Hilfe einer Schneidevorrichtung (16) geschnitten werden.

## Revendications

1. Couche-culotte jetable de type short qui comprend une feuille supérieure perméable au liquide (2), une feuille arrière imperméable au liquide (3) et un noyau absorbant qui retient le liquide (4), et qui présente une partie d'ouverture pour la taille (D) et une paire de parties d'ouverture pour les jambes (E, E) ;
ladite partie ouverture pour une jambe (E) présentant une région élastique de jambe (70) formée de manière annulaire le long d'un bord périphérique de celle-ci, ladite région élastique de jambe (70) comprenant une pluralité de premiers éléments élastiques (71) agencés à partir d'une partie côté estomac (A) vers une partie entrejambe (C) et une pluralité de seconds éléments élastiques (72) agencés à partir d'une partie côté arrière (B) vers la partie entrejambe (C) ;
lesdits premiers et seconds éléments élastiques (71, 72) comprenant une partie formation de partie annulaire (71a, 72a) destinée à former ladite région élastique de jambe (70) et une partie extension (71b, 72b) qui s'étend à partir de ladite partie formation de partie annulaire (71 a, 72a) vers le centre dans le sens de la largeur de ladite couche-culotte, lesdites parties extensions respectives (71b, 72b) desdits premiers et seconds éléments élastiques (71, 72) comprenant une partie extrémité (71c, 72c) au niveau d'un endroit où lesdites parties extensions (71b, 72b) n'atteignent pas le centre dans le sens de la largeur de ladite couche-culotte, lesdites parties extension (71 b, 72b) étant conçues de façon à ne pas présenter d'aptitude à une contraction élastique à proximité de ladite partie extrémité (71c, 72c), lesdites parties extensions (71b, 72b) desdits premiers et seconds éléments élastiques (71, 72) étant agencées de telle sorte que des paires d'un premier élément élastique (71) et d'un second élément élastique (72) se chevauchent, dans laquelle les parties où ledit premier élément élastique (71) et ledit second élément élastique (72) se chevauchent les uns les autres, respectivement, forment les parties extensions (71b, 72b) des éléments élastiques respectifs (71, 72).

2. Couche-culotte jetable de type short selon la revendication 1, dans laquelle lesdites parties extensions (71b, 72b) desdits premiers et seconds éléments élastiques (71, 72) se trouvent dans une position qui se situe sur un côté de la partie côté estomac à partir du centre longitudinal de ladite couche-culotte dans un état où la couche-culotte du type short est déployée et étirée.

3. Couche-culotte jetable de type short selon la revendication 1 ou la revendication 2, dans laquelle lesdits premiers et seconds éléments élastiques (71, 72) sont fixés, dans ladite région élastique de jambe (70), à un matériau de feuille adjacent par des premiers moyens de fixation et sont fixés, à proximité (M) d'une frontière entre ladite partie formation de partie annulaire (71a, 72a) et ladite partie extension (71b, 72b), au matériau de feuille adjacent par des seconds moyens de fixation qui sont appliqués à une plage plus étroite que la plage à laquelle lesdits premiers moyens de fixation sont appliqués, ou par lesdits premiers moyens de fixation et lesdits seconds moyens de fixation.

4. Couche-culotte jetable de type short selon la revendication 3, dans laquelle lesdits premiers et seconds éléments élastiques (71, 72) sont fixés à deux matériaux de feuille adjacents à proximité d'une frontière entre ladite partie formation de partie annulaire (71a, 72a) et ladite partie extension (71 b, 72b).

5. Couche-culotte jetable de type short selon la revendication 1, dans laquelle lesdites parties extension (71b, 72b) desdits premiers et seconds éléments élastiques (71, 72) se chevauchent au moins au niveau d'une partie de celles-ci avec ledit noyau absorbant (4).

6. Couche-culotte jetable de type short selon la revendication 1, dans laquelle lesdits premiers et seconds éléments élastiques (71, 72) sont pris en sandwich dans leurs états étirés entre deux feuilles qui sont formées de manière séparée à partir de ladite feuille arrière (3) et disposées sur un côté de surface extérieure de ladite feuille arrière (3).

7. Procédé de fabrication d'une couche-culotte jetable de type short présentant des premier et second éléments élastiques (71, 72) qui sont agencés le long des bords périphériques d'une paire de parties ouverture de jambe (E, E) et qui ne sont pas agencés au niveau d'une partie centrale dans le sens de la largeur d'une partie entrejambe (C), ledit procédé comprenant les étapes consistant à :
introduire lesdits premiers et seconds éléments élastiques (71, 72) dans leurs états étirés sur une feuille de matériau de base (11) qui est transportée de manière continue tout en agitant lesdits premiers et seconds éléments élastiques (71, 72) dans une direction orthogonale à une direction d'écoulement de ladite feuille de matériau de base (11), et couper ensuite lesdits premiers et seconds éléments élastiques (71, 72) au niveau d'une partie centrale d'entrejambe (C') qui correspond à une partie centrale dans le sens de la largeur d'une partie entrejambe (C) de ladite couche-culotte ;
lesdits premiers et seconds éléments élastiques (71, 72) étant introduits dans un état où un premier élément élastique (71) et un second élément élastique (72) se chevauchent l'un l'autre au niveau de la partie centrale d'entrejambe (C') et lesdits éléments élastiques (71, 72) qui se chevauchent étant pressés et coupés dans une direction dans le sens de l'épaisseur par une saillie de coupe (16).
